# EUROPÄISCHE PATENTSCHRIFT

(11) **EP 0 410 306 B1**
(45) Veröffentlichungstag und Bekanntmachung des Hinweises auf die Patenterteilung: **11.01.1995**
(21) Anmeldenummer: 90113871.9
(22) Anmeldetag: 19.07.1990
(51) Int. Cl.: A61L 11/00, A61L 2/12

(54) **Verfahren und Vorrichtung zur Sterilisation von Müll, insbesondere Krankenhausmüll**
Process and apparatus for the sterilisation of waste, especially hospital waste
Procédé et dispositif pour la stérilisation de déchets, notamment de déchets d'hôpitaux

(30) Priorität: 26.07.1989 DE 3924744
(43) Veröffentlichungstag der Anmeldung: 30.01.1991
(73) Patentinhaber: NORDPUNKT AG, CH-6986 Novaggio/TI (CH)
(72) Erfinder: Maihofer, Willi, D-7460 Balingen/Weilstetten (DE)
(74) Vertreter: Schwabe - Sandmair - Marx

(56) Entgegenhaltungen:
- WO-A-90/12601
- DE-A- 2 908 086
- DE-A- 3 317 300
- DE-C- 3 505 570

## Beschreibung

Die Erfindung betrifft ein Verfahren und eine Vorrichtung zum Sterilisieren von Müll, insbesondere Krankenhausmüll, unter Verwendung von mehreren Einweg-Müllbehältern, die nach Beladung mit Müll durch einen Deckel hermetisch verschlossen werden und dann vorzugsweise zu Mehreren in einem Sterilisierungstunnel eingebracht werden und anschließend mit Mikrowellen bestrahlt werden, wobei entweder vor oder während der Mikrowellenbestrahlung Wasser oder ein Desinfektionsmittel in den Müll der Müllbehälter eingebracht wird und der oder die Müllbehälter dann an einem Austragende aus dem Sterilisierungstunnel ausgetragen werden.

Aus der DE-OS 33 17 300 ist ein Behälter zur Aufnahme von Infektionsmüll, insbesondere von Krankenhausabfall, bekannt. Zur Verhütung von Infektionen wird dieser Müll mit Hilfe von Mikrowellen sterilisiert, was dadurch möglich ist, daß der von außen verschließbare Behälter aus einem Material besteht, durch welches Mikrowellen hindurch gelangen können. Dieser Behälter ist in seinem Inneren mit einem einsetzbaren, standfesten und verschließbaren Einmal-Innenbehälter für den Infektionsmüll ausgestattet, der aus einem im wesentlichen feuchtigkeitsdichten, sowie Dampf und/oder Mikrowellen jedoch hindurchlassenden reißfestem Material besteht. Der Außenbehälter dieser Anordnung kann als Druckbehälter ausgeführt sein.

An der Innenseite des Deckels des genannten Innenbehälters kann ein Beutel oder dergleichen mit Flüssigkeit, vorzugsweise Desinfektionsmittel, angebracht werden. Während der Mikrowellenbehandlung wird die Flüssigkeit im Inneren des Beutels erhitzt, verdampft teilweise, wodurch der Beutel schließlich platzt und sein Inhalt in den umgebenden Müll entleert wird.

Aus der EP 0 049 430 ist ein Massengutbehälter mit einem Deckel, vorzugsweise zur Verwendung als Einweg-Transportgefäß, bekannt, der auch für die Aufnahme von Krankenhausmüll eingesetzt werden kann.

Aus der DE-OS 29 08 086 ist ein Verfahren zum Desinfizieren und Sterilisieren von mit Keimen behafteten Gegenstände bekannt, wobei dieses bekannte Verfahren darin besteht, daß im Raum um den zu behandelnden Gegenstand ein Desinfektionsmittel vernebelt wird. Dieses bekannte Verfahren kann ferner dadurch weiter ausgestaltet werden, daß der von dem Desinfektionsmittel umgebene Gegenstand mit Mikrowellen bestrahlt wird. Der Desinfizierungsvorgang erfolgt in einer abgeschlossenen und abgedichteten Desinfektionskammer, wobei innerhalb der Desinfektionskammer ein Vernebelungsgerät für Desinfektionsmittel angeordnet ist. Eine weitere Ausgestaltung dieser Desinfektionskammer besteht darin, daß in derselben ein Mikrowellengerät und außerhalb der Desinfektionskammer ein Steuergerät vorgesehen ist, mit welchem zunächst das Vernebelungsgerät und erst nach Ausbildung eines Desinfektionsnebels in der Desinfektionskammer das Mikrowellengerät eingeschaltet wird.

Bei einer derartigen Desinfektionskammer, die auch in Form eines Sterilisierungstunnels aufgebaut sein kann, wobei an den Wänden des Tunnels aufeinanderfolgend mehrere Mikrowellengeneratoren angeordnet sind, besteht jedoch die Möglichkeit, wenn der Müll unmittelbar in die Kammer eingebracht wird, daß Keime aus dieser Kammer oder Tunnel entweichen können, beispielsweise bei Entstehen eines Durchzuges, d. h. die zu tötenden Keime oder Bakterien können zufällig nicht ausreichend lang in der Kammer oder in dem Tunnel gehalten werden, so daß eine 100 %ige Sterilisierung und Desinfektion nicht mit großer Sicherheit erreicht werden kann.

Aus der DE 37 10 156 A1 ist eine Vorrichtung zur Behandlung von Infektionsmüll mit Hilfe von Mikrowellen bekannt. Die bekannte Vorrichtung umfaßt eine Mikrowellenkammer, in die mit dem Infektionsmüll gefüllte Behälter, die mit Hilfe eines Deckels hermetisch verschlossen werden, eingebracht werden. Die Behälter besteht aus mikrowellendurchlässigem Material und in der Mikrowellenkammer selbst ist eine Einrichtung zur Bewegung der Behälter während der Bestrahlung mit Mikrowellen vorgesehen. Diese Einrichtung kann beispielweise ein Förderband oder ein Drehtisch sein.

Die Behälter selbst können im Inneren einen weiteren Behälter zur Aufnahme einer Flüssigkeit, insbesondere Wasser, aufweisen.

Aus der DE 35 05 571 C2 ist eine Vorrichtung zur Desinfektion und Sterilisation von Matratzen oder dergleichen mit Hilfe von Mikrowellen bekannt, die eine Mikrowellenkammer zur Aufnahme der Matratzen und mindestens einen Mikrowellensender zum Bestrahlen der angefeuchteten Matratzen aufweist. Das wesentliche dieser bekannten Vorrichtung besteht darin, daß die allseitig abgeschlossene Mikrowellenkammer mikrowellendurchlässige Fenster aufweist, durch die Mikrowellen in die Mikrowellenkammer gestrahlt werden, daß innerhalb der Mikrowellenkammer eine mikrowellendurchlässige Matratzenkammer aufgenommen ist, und daß Mikrowellenkammer und Matratzenkammer miteinander kommunizieren, wobei die Mikrowellenkammer gemeinsam mit der Matratzenkammer als Schleuse ausgebildet ist und zu beiden Seiten der Matratzenkammer Schleusentüren vorgesehen sind.

Für die Entsorgung von infektiösem Krankenhausmüll gibt es eine Reihe von Vorschriften, deren Einhaltung vom Bundesgesundheitsamt zu überwachen sind, welches hierzu auch eine Verordnung erlassen hat. Der Grundgedanke ist hierbei, daß bei der Entsorgung von infektiösem Krankenhausmüll keine Kontaminierung der Umgebung auftreten darf.

Bei der Mikrowellenbehandlung von infektiösem Krankenhausmüll ergeben sich in Verbindung mit den fest verschlossenen Behälern jedoch insofern Schwierigkeiten, als in die Kunststoffbehälter Wasser eingegeben werden muß, damit überhaupt eine Erhitzung des an sich verhältnismäßig trockenen Krankenhausmülls möglich ist. Man könnte zum Beispiel auch Wasser in Form von verschlossenen Kunststoffbeuteln zugeben, die die betreffende Bedienungsperson nach dem Befüllen der Kunststoffbehälter diesen noch beigibt. Eine solche Lösung wäre aber von Seiten der Sicherheit her nicht akzeptierbar, weil dabei nicht sichergestellt werden kann, daß die Bedienungsperson gelegentlich die Beigabe eines Wasserbehälters in den Kunststoffbehälter vergißt und dann keine ausreichende Erhitzung und Sterilisierung des Krankenhausmülls möglich ist.

Auch dann, wenn man von vornherein in den Behältern Bereiche vorsieht, in denen Wasser aufbewahrt wird, welches während der Mikrowellenbehandlung freigesetzt wird, damit der Müll im Inneren des Behälters mit Wasser benetzt wird, bietet keine ausreichende Sicherheit, da die betreffende Bedienungsperson auch einmal vergessen kann zu überprüfen, ob ein verwendeter Behälter tatsächlich noch ein unbeschädigtes Wasserreservoir enthält.

Es muß daher sichergestellt werden, daß das Wasser unabhängig von der Aufmerksamkeit der Bedienungsperson immer zuverlässig in die betreffenden Behälter gelangt.

Ein weiteres Problem bei der Erwärmung des infektiösen Krankenhausmülls in Kunststoffbehältern besteht darin, daß durch die Erhitzung in diesen ein Überdruck entsteht, welcher den Behälter zum Platzen bringen könnte.

Die der Erfindung zugrundeliegende Aufgabe besteht darin, ein Verfahren und eine Vorrichtung zur Sterilisation von Müll, insbesondere Krankenhausmüll der angegebenen Gattung, zu schaffen, bei dem bzw. bei der die Sterilisierung mit sehr hoher Sicherheit durchgeführt werden kann und insbesondere immer gewährleistet wird, daß Wasser unabhängig von der Aufmerksamkeit einer Bedienungsperson in den betreffenden Müllbehälter gelangt.

Diese Aufgabe wird erfindungsgemäß dadurch gelöst, daß
a) die mit Müll beladenen und mit einem Deckel hermetisch verschlossenen Müllbehälter auf ein Vorbereitungsförderband aufgebracht werden, welches in einer Schleusenkammer des Sterilisierungstunnels angeordnet ist,
b) die Schleusenkammer dann hermetisch am Eingabeende verschlossen wird,
c) eine Schleusentür innerhalb des Sterilisierungstunnels geöffnet wird und die Müllbehälter von dem Vorbereitungsförderband auf ein Hauptförderband innerhalb einer Sterilisierungskammer aufgebracht werden,
d) die Sterilisierungskammer dann hermetisch verschlossen wird und über bewegbar in der Sterilisierungskammer angeordnete Injektionsnadeln Wasser oder Desinfektionsmittel in jeden einzelnen Müllbehälter injiziert wird, und
e) nach der Mikrowellenbehandlung alle in der Sterilisierungskammer vorhandenen Müllbehälter aus der Sterilisierungskammer am Austragende mit Hilfe des Hauptförderbandes ausgetragen werden, wobei die Schleusentür hermetisch verschlossen gehalten wird.

Das Verfahren nach der vorliegenden Erfindung kann unter Gewährleistung einer hohen Sicherheit gegenüber einer Kontaminierung der unmittelbaren Umgebung der Sterilisierungsanlage durchgeführt werden, wobei auch immer sichergestellt wird, daß Wasser oder ein Desinfektionsmittel im Inneren der Müllbehälter vorhanden ist.

Die Erfindung kann dadurch eine vorteilhafte Ausgestaltung erfahren, daß die Injektionsnadeln in der Sterilisierungskammer von oben nach unten auf den jeweiligen Müllbehälter abgesenkt werden, um den jeweiligen Deckel der Müllbehälter zu durchstechen.

Es besteht jedoch auch die Möglichkeit, die Injektionsnadeln in der Sterilisierungskammer von der Seite her auf die Müllbehälter zu zu bewegen, um die Müllbehälter seitlich zu durchstechen.

Um das Austreten von Krankheitskeimen aus dem Inneren des jeweiligen Müllbehälters mit hoher Sicherheit zu verhindern, werden die Injektionsnadeln während der Mikrowellenbehandlung in ihrem Einstichzustand in Lage festgehalten.

Eine vorteilhafte Ausgestaltung der Erfindung besteht ferner darin, daß mehr als eine Injektionsnadel pro Müllbehälter eingestochen wird. Dabei besteht die Möglichkeit, eine der Injektionsnadeln dazu zu verwenden, um Wasser oder ein Desinfektionsmittel in den Müllbehälter einzuleiten, während eine zweite Nadel dazu verwendet werden kann, um Luft oder irgendwelche Gase innerhalb des Müllbehälters abzuleiten, um die Entstehung eines Überdrucks innerhalb des Müllbehälters zu verhindern. Das dabei abgesaugte Gas kann über einen Sterilisierungskreislauf geschickt werden und dann entweder in die Atmosphäre abgegeben werden oder in die Sterilisierungskammer eingeleitet werden.

Ferner besteht auch die Möglichkeit, wenigstens eine der Injektionsnadeln dazu zu verwenden, um vorzugsweise erhitzten Wasserdampf in den Müllbehäler zu injizieren. Das aus dem jeweiligen Müllbehälter abgesaugte Gas kann über ein Filter, einen Erhitzungsbereich und/oder einen Mikrowellenstrahlungsbereich geleitet werden, um es ebenfalls zu sterilisieren.

Um auch das Ausdringen von Keimen aus der Sterilisierungskammer und aus der Schleusenkammer wirksam zu verhindern, wird die Schleusentür zwischen der Schleusenkammer und der Sterilisierungskammer hermetisch verriegelt, wenn die Austragtür der Sterilisierungskammer oder die Eintragtür der Schleusenkammer geöffnet wird und ferner wird die Schleusentür nur dann entriegelt, wenn die Austragtür der Sterilisierungskammer geschlossen ist und die Eintragtür der Schleusenkammer geschlossen wird.

Die Erfindung betrifft ferner auch eine Vorrichtung zur Durchführung des erfindungsgemäßen Verfahrens, die einen Sterilisierungstunnel umfaßt, der eine Sterilisierungskammer mit einem Eintragende und einem Austragende und einem Förderband aufweist.

Die Erfindung betrifft ferner auch eine Einrichtung zur Sterilisierung von Müll, insbesondere Krankenhausmüll, mit mehreren durch einen Deckel verschließbaren und von einer Injektionsnadel durchstoßbaren Müllbehältern, die für die Sterilisierung von in ihnen enthaltenem Müll in einem Mikrowellen-Sterilisierungstunnel geeignet sind, welcher wenigstens eine Förderbahn zum gleichzeitigen Fördern von mehreren hintereinander angeordneten Müllbehältern enthält und welcher eine Injektionseinrichtung aufweist, um in einer Reihe von Müllbehältern vorzugsweise gleichzeitig Wasser oder ein Desinfektionsittel zu injizieren.

Erfindungsgemäß ist diese Einrichtung zur Sterilisierung von Müll, insbesondere Krankenhausmüll, so ausgeführt, daß sie in besonders gedrängter Bauweise ausgeführt werden kann und immer sichergestellt wird, daß der Einstich der jeweiligen Injektionsnadel in den Behälter an einer genau definierten Soll-Einstichstelle erfolgt, um dadurch eine größere Sicherheit bei der Abdichtung des Müllbehältrs zu erreichen.

Gemäß dieser Ausführungsform ist wenigstens eine Förderbahn aus einer Rollenförderbahn gebildet, die vom Eintragende zum Austragende des Sterilisierungstunnel hin abwärts geneigt ist, so daß dadurch die auf die Rollenförderbahn aufgesetzten oder eingehängten Müllbehälter aufgrund ihres Eigengewichtes in Richtung zum Austragende des Sterilisiserungstunnels befördert werden.

Die Müllbehälter sind jeweils in der Nähe ihrer Oberseite mit einem umlaufenden Flansch versehen, mit dessen Hilfe die Müllbehälter auf die wenigstens eine Rollenförderbahn aufgesetzt und von dieser getragen werden. Schließlich sind die Müllbehälter jeweils mit eine Soll-Einstichstelle ausgestattet, die aus einem den Einstich einer Injektionsnadel verschließenden Wandabschnitt besteht.

Um einen hohen Wirkungsgrad bei der Sterilisierung von Müll zu erreichen, kann die Erfindung dadurch eine vorteilhafte Ausgestaltung erfahren, daß in dem Sterilisierungstunnel zwei Rollenförderbahnen parallel nebeneinander verlaufend angeordnet sind.

Durch diese letztere Maßnahme wird die gesamte Baulänge des Sterilisierungstunnels nicht erhöht und es kann trotzdem eine gedrängte kurze Baulänge erreicht werden.

Zweckmäßigerweise ist am Austragende der jeweiligen Rollenförderbahn ein lösbarer Anschlag vorgesehen, bis zu welchem der erste in den Sterilisierungstunnel eingetragene Müllbehälter rollen kann. Die Neigung der jeweiligen Rollenförderbahn ist dabei derart bemessen, daß die in der jeweiligen Rollenförderbahn aneinander gereihten Müllbehälter aufgrund ihres Eigengewichtes in Richtung zum Austragende des Sterilisierungstunnels rollen, wobei der vorderste Müllbehälter an dem lösbaren Anschlag abgestützt wird und sich die nachfolgenden Müllbehälter aneinander abstützen, so daß dadurch eine durchgehende nicht unterbrochene Behälterreihe gebildet wird.

Durch dieses letztere Merkmal wird u. a. auch der Vorteil erreicht, daß die Müllbehälter auf der jeweiligen Rollenförderbahn exakt relativ zueinander positioniert werden, so daß der Einstich der jeweiligen Injektionsnadeln an einer genau definierten Stelle des Behälters vorgenommen werden kann.

Zum Weitertransport der behandelten Müllbehälter kann ferner ein Querförderband am Austragende des Sterilisierungstunnels angeordnet sein, um die aus dem Sterilisierungstunnel ausgetragenen Müllbehälter dann zu einem Preßmüllcontainer zu fördern.

Zur Erhöhung der Sicherheit kann ferner am Eintragende des Sterilisierungstunnels eine kleine Schleusenkammer angeordnet sein.

Der Sterilisierungstunnel kann selbst verfahrbar angeordnet sein und beispielsweise auf einem Lastwagen oder Lastwagenanhänger installiert sein.

Die Müllbehälter sind in vorteilhafter Weise in Draufsicht etwa quadratisch oder rechteckig gestaltet und besitzen abgerundete Ecken.

Dadurch wird eine Fehlausrichung der Müllbehälter bei der Beladung eines Sterilisierungstunnels weitgehend ausgeschaltet.

Der umlaufende Flansch des jeweiligen Müllbehälters weist in Abständen voneinander angeordnete Durchbrüche auf, durch welche am Deckel des Müllbehälters ausgebildete Verriegelungszungen einschiebbar sind, um den Deckel an dem umlaufenden Flansch zu verriegeln. Der umlaufende Flansch hat damit zwei verschiedene Funktionen, und zwar dient er zum einen dazu, den Müllbehälter auf der jeweiligen Rollenförderbahn abzustützen und zu halten und dient zum anderen dazu, einen sicheren Verschluß zwischen Deckel und Müllbehälter zu erreichen.

Um eine besonders sichere Abdichtung zwischen Deckel und Behälter zu erreichen, weist der Deckel eine den oberen Behälterrand aufnehmende umlaufende Nut auf, in der ein Dichtungselement zur Abdichtung des Deckels am Behälterrand angeordnet ist. Dieses Dichtungselement beseht in bevorzugter Weise aus einem elastischen Material, wie beispielsweise Gummi, so daß dadurch der Verriegelungsvorgang problemlos und schnell vorgenommen werden kann.

Im einzelnen kann die Erfindung ferner dadurch noch eine vorteilhafte Ausgestaltung erfahren, daß der die Soll-Einstichstelle festlegende Wandabschnitt am Deckel des Müllbehälters ausgebildet ist und aus einer sich keilförmig verengenden Wandvertiefung besteht, wobei die Berührungslinie der keilförmig verlaufenden Wände am Boden der Vertiefung die Einstichstelle bildet. Die am Boden der Vertiefung sich berührenden Wandabschnitte sind einstückig miteinander verbunden, so daß dieser Bereich vollkommen dicht ist und irgendwelche Gase aus dem Inneren des Müllbehälters dort nicht entweichen können. Die keilförmig zusammenlaufenden Wandabschnitte gehen in vorteilhafter Weise in einen verlängerten Wandfortsatz über, der zum Inneren des Behälters hinweist. Der Wandfortsatz kann zweckmäßigerweise in einen verdünnten Wandfortsatzrand auslaufen, wodurch eine ganz spezifische Abdichtwirkung erreicht wird. Wenn nämlich eine Injektionsnadel durch den Boden der Vertiefung eingestochen wird, und nach Einleitung von Wasser in den Behälter diese Injektionsnadel wieder herausgezogen wird, so wird durch den im Inneren des Behälters sich allmählich aufbauenden Druck die Einstichstelle wirksam wieder verschlossen, wobei die die Einstichstelle verschließende Kraft umso größer ist, je höher der Druck im Inneren des Behälters ist.

Um eine besonders wirksame Durchmengung des Wassers mit dem Müll innerhalb des jeweiligen Müllbehälters zu erreichen, kann ferner im Boden des jeweiligen Müllbehälters eine als Wasserreservoir dienende Vertiefung ausgebildet sein.

Um den Verlauf des Sterilisierungsvorganges zu überwachen, kann ferner jede der Injektionsnadeln mit einer Temperatur- und Druckmeßeinrichtung verbunden sein. Die Druckmeßeinrichtung kann beispielsweise in dem Leitungssystem der Wasserversorgung integriert sein. Dabei kann die Druckmeßeinrichtung zentral am Wasserverteilungssystem vorgesehen sein, wobei eine Wasserhauptleitung vorhanden ist, von der sich die einzelnen Wasserzuleitungen zu den Injektionsnadeln abzweigen.

Eine weitere, besonders vorteilhafte Ausgestaltung der Erfindung besteht darin, daß die Injektionsnadel selbst aus einem Temperaturfühler besteht bzw. als Temperaturfühler ausgebildet ist. Bei dieser letzteren Ausführungsform kann die Injektionsnadel daher zwei verschiedene Funktionen erfüllen.

Eine weitere Ausführungsform besteht ferner darin, daß in dem Hohlraum der Injektionsnadel eine Temperaturmeßsonde verschiebbar angeordnet ist. Diese Temperaturmeßsonde kann zweckmäßigerweise bei einer Temperaturmessung einige Zentimeter über die Nadelspitze herausgeschoben werden, wenn die Nadel in einen Behälter eingestochen ist, und zwar so weit, daß die Temperatur gut gemessen werden kann, jedoch dabei trotzdem nicht die Gefahr besteht, daß die Temperaturmeßsonde auf Müll auftrifft und beschädigt wird.

Der Deckel und/oder der Behälter können zweckmäßigerweise aus Polyethylen oder Polypropylen bestehen.

Besonders vorteilhafte Ausgestaltungen und Weiterbildungen der erfindungsgemäßen Vorrichtung und Einrichtung ergeben sich aus den Ansprüchen 11 bis 45.

Im folgenden wird die Erfindung anhand eines Ausführungsbeispiels unter Hinweis auf die Zeichnung näher erläutert. Es zeigen:
- Fig. 1: eine Seitenansicht, teilweise in weggebrochener Darstellung, einer Sterilisierungsanlage in Form eines Containers zur Durchführung des erfindungsgemäßen Verfahrens;
- Fig. 2: eine Draufsicht auf die Sterilisierungsanlage von Fig. 1;
- Fig. 3: eine schematische Seitenansicht eines Sterilisierungstunnels mit Merkmalen nach der Erfindung;
- Fig. 4: eine Schnittdarstellung des Sterilisierungstunnels gemäß der Linie I-I in Fig. 3; und
- Fig. 5: eine schematische Schnittdarstellung eines Teiles des Müllbehälters und des zugeordneten Deckels.

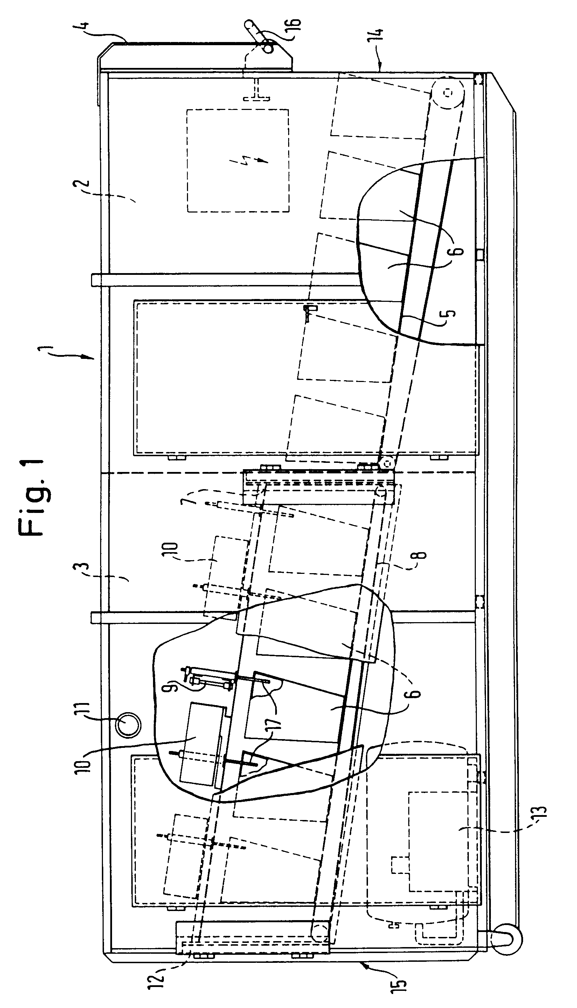

Fig. 1 zeigt eine Seitenansicht, teilweise in gebrochener Darstellung, einer Sterilisierungsanlage in Form eines Containers, der allgemein mit 1 bezeichnet ist. Der Container 1 ist bei dem gezeigten Ausführungsbeispiel an keinem festen Platz angeordnet, sondern ist transportierbar und kann daher z. B. auf einen Lastwagen aufgestellt werden oder kann sogar einen Lastwagenanhänger selbst bilden. In diesem Container 1 ist die Anlage zur Sterilisierung von infektiösem Krankenhausmüll untergebracht. Die gezeigte Anlage weist ein Eingabeende 14 auf, welches durch eine verschwenkbar angeordnete Tür 4 verschlossen ist. Diese Tür 4 kann mit Hilfe eines Handgriffes 16 von einer Bedienungsperson geöffnet und geschlossen werden. Im Inneren des Containers sind grundsätzlich zwei Kammern ausgebildet, und zwar eine erste Schleusenkammer 2 und eine nachfolgende Sterilisierungskammer 3.

Innerhalb der Schleusenkammer 2 ist ein Vorbereitungsförderband 5 angeordnet, welches derart bemessen ist, daß auf demselben mehrere Kunststoffbehälter, beispielsweise fünf Kunststoffbehälter in geringem Abstand zueinander in Förderrichtung des Bandes hintereinander angeordnet werden können. Ferner ist das Vorbereitungsförderband 5 beispielsweise so breit bemessen, daß zwei oder mehr Kunststoffbhälter 6 nebeneinander auf dem Förderband angeordnet werden können. Bei dem gezeigten Ausführungsbeispiel verläuft das Vorbereitungsförderband 5 in Förderrichtung schräg nach oben, so daß das Eintragsende der Schleusenkammer 2 niedriger angeordnet werden kann als das Austragsende. Die Schleusenkammer 2 ist mit Hilfe einer Schleusentür 7 gegenüber der Sterilisierungskammer 3 hermetisch abdichtbar und es ist ein Mechanismus (nicht gezeigt) vorgesehen, der bewirkt, daß die Schleusentür 7 immer hermetisch verschlossen bleibt, so lange entweder die Tür 4 am Eintragsende 14 geöffnet ist oder die Tür 12 am Austragsende 15 Sterilisierungskammer 3 geöffnet ist. Dadurch wird sichergestellt, daß beispielsweise während der Mikrowellenbehandlung Gase innerhalb der Sterilisierungskammer nicht in die Schleusenkammer 2 eindringen können und umgekehrt.

Ferner kann auch eine Einrichtung vorgesehen werden, durch die bewirkt wird, daß nach der Überführung der Müllbehälter 6 von dem Vorbereitungsförderband 5 auf ein Hauptförderband innerhalb der Sterilisierungskammer 3 die Austragstür 12 solange geschlossen gehalten wird, bis der Sterilisierungsvorgang vollständig abgeschlossen ist.

Wie dies aus Fig. 1 zu erkennen ist, schließt sich an die Schleusenkammer 2 unmittelbar die Sterilisierungskammer 3 an und nach Öffnen der Schleusentür 7 kann die Ladung an Kunststoffbehältern 6, die auf dem Vorbereitungsförderband 5 steht, auf den Hauptförderer 8 überführt werden.

Wenn dann diese Charge an Kunststoffbehältern 6 innerhalb der Sterilisierungskammer 3 angelangt ist, wird die Schleusentür 7, die beispielsweise als Schiebetür ausgebildet sein kann, hermetisch verschlossen und ebenso wird die Austragstür 12 am Austragsende 15 der Sterilisierungskammer 3 hermetisch verschlossen. Es werden dann mit Hilfe eines Mechanismus 10 Injektionsnadeln von oberhalb der Kunststoffbehälter 6 abgesenkt, so daß die Injektionsnadeln 17 den jeweils unter ihnen angeordneten Kunststoffbehälter im Deckelbereich durchstechen. Über die Injektionsnadeln 17 kann dann in das Innere des jeweiligen Behälters Wasser oder ein Desinfektionsmittel eingespritzt werden.

Um das Platzen der jeweiligen Kunststoffbehälter durch das Injizieren von Wasser oder Desinfektionsmittel und aufgrund der nachfolgenden Erhitzung des befeuchteten infektiösen Krankenhausmülls innerhalb der Behälter 6 zu verhindern, kann entweder in die Sterilisierungskammer 3 über eine geeignete Einrichtung und eine Öffnung 11 beispielsweise heißer Wasserdampf eingeleitet werden, so daß sich im Inneren der Sterilisierungskammer 3 ein entsprechend hoher Gegendruck aufbaut. Alternativ besteht jedoch auch die Möglichkeit, dem jeweiligen Kunststoffbehälter 6 mehrere Injektionsnadeln zuzuordnen, d. h. der jeweilige Deckel eines Kunststoffbehälters wird beispielsweise von zwei in geringem Abstand voneinander angeordneten Injektionsnadeln durchstochen, wobei eine Injektionsnadel dafür vorgesehen ist, um Wasser oder das Desinfektionsmittel in den betreffenden Behälter einzuleiten, während die andere Nadel dazu dient, um Gase innerhalb des jeweiligen Kunststoffbehälters abzusaugen und über einen getrennten (nicht gezeigten) Kreislauf zu sterilisieren. Dieser Kreislauf kann geeignete Filtereinrichtungen, wie z. B. Aktivkohlefilter oder dergleichen, enthalten oder er kann Abschnitte enthalten, an denen das über die Nadel abgesaugte Gas auf eine hohe Temperatur erhitzt wird, um dadurch eine Sterilisierung zu erreichen. Das sterilisierte Gas kann dann entweder in den Sterilisierungsraum 3 zurückgeleitet werden oder anderweitig vernichtet bzw. in die Atmosphäre abgeleitet werden.

Die in den jeweiligen Kunststoffbehältern eingestochenen Injektionsnadeln bleiben in diesem Zustand so lange eingestochen, bis der gesamte Sterilisierungsvorgang abgeschlossen ist. Dadurch wird verhindert, daß über die Injektionsnadeln Krankheitskeime aus dem Inneren der jeweiligen Kunststoffbehälter nach außen dringen können. Darüber hinaus dienen die Injektionsnadeln auch dazu, um den jeweiligen Kunststoffbehälter 6 in Lage zu halten.

Während eines Sterilisierungsvorganges kann das Hauptförderband 8 über einen Antriebsmechanismus 13 so angetrieben werden, daß die Behälter auf dem Hauptförderband 8 hin- und herbewegt werden. Diese hin- und hergehende Bewegung wird von den Injektionsnadeln 17 und den zugeordneten Einrichtungen 10 mit ausgeführt.

Nach der Mikrowellenbehandlung werden schließlich die Injektionsnadeln 17 mit Hilfe geeigneter Antriebsmittel 9 aus den jeweiligen Deckelteilen herausgezogen und es wird danach das Austragsende 15 über die Tür 12 geöffnet. Das Hauptförderband 8 wird dann entsprechend angetrieben, um die behandelten Kunststoffbehälter aus der Anlage auszutragen.

Es sei an dieser Stelle darauf hingewiesen, daß es sich bei den Behältern 6 einerseits um Kunststoffbehälter handelt, die für Mikrowellen durchlässig sind, andererseits jedoch auch um Einwegbehälter, die beispielsweise mit Hilfe eines Preßmüllcontainers beseitigt werden, der sie mit 30 Tonnen Druck unter Volumenreduzierung zusammenpreßt. Dabei dürfen die jeweiligen Behälter auch platzen, da sie ja nunmehr nur noch sterilen Müll enthalten.

Gleichzeitig kann natürlich eine neue Charge von zehn Behältern auf das Vorbereitungsförderband in die Schleusenkammer 2 eingefahren werden.

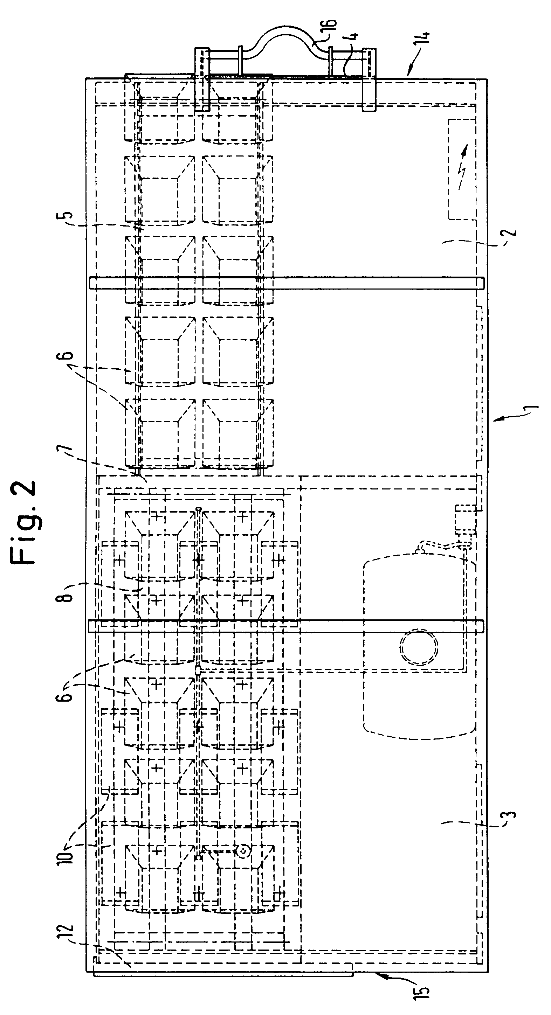

Bei einer praktischen Realisierung der in Fig. 1 und 2 gezeigten Anlage wird das Vorbereitungsförderband 5 schrittweise beim Beladungsvorgang bewegt, bis schließlich insgesamt zehn Müllbehälter 6 auf dem Vorbereitungsförderband 5 angeordnet sind. Danach wird as Eintragsende 14 über die Tür 4 verschlossen und es wird die Charge innerhalb der Schleusenkammer 2 in die Sterilisierungskammer 3 in der geschilderten Weise überführt.

Die vorliegende Erfindung ist jedoch nicht auf das Ausführungsbeispiel gemäß den Fig. 1 bis 2 beschränkt.

Es sind für einen Fachmann eine Reihe von Abwandlungen und Änderungen bei diesem Ausführungsbeipsiel möglich. So besteht beispielsweise die Möglichkeit, anstelle des Vorbereitungsförderbandes auch einen Drehtisch vorzusehen, was auch für das Hauptförderband 8 glt und es besteht ferner die Möglichkeit, die Anlage mit einem Codeleser auszustatten, der einen bestimmten Code, welcher an den Behältern 6 angebracht ist, zu lesen, um dadurch festzustellen, ob es sich bei dem betreffenden Müllbehältr um einen für die Sterilisierungsanlage geeigneten Behälter handelt und um nicht geeignete Müllbehälter abzuweisen, so daß dadurch die Sicherheit noch beträchtlich erhöht wird.

Ferner besteht die Möglichkeit, sämtliche Türen der Anlage als Schiebetüren auszubilden und ferner ist für den Fachmann auch ohne weiteres erkennbar, daß ein Verriegelungsmechanismus für jede der Türen so ausgebildet werden kann, daß bei bestimmten Türstellungskombinationen bestimmte Verriegelungs- und/oder Entriegelungszustände an anderen Türen eintreten. Dadurch kann in jedem Fall sichergestellt werden, daß unbehandelte Müllbehälter die Anlage nicht ohne eine Sterilisation verlassen können.

Auch ist die vorliegende Erfindung offensichtlich nicht auf die Zahl von zehn Müllbehältern, die eine Charge ausmachen, beschränkt.

Außerdem besteht auch die Möglichkeit, die Injektionsnadeln nicht von oben nach unten in die Kunststoffbehälter 6 bzw. die Deckelelemente derselben einzustechen, sondern die Injektionsnadeln seitlich bzw. in horizontaler Richtung in die jeweiligen Behälter einzustechen.

Die Kunststoffbehälter können auch mit einer Öffnung versehen sein, die bei der Erwärmung des Inhalts der Kunststoffbehälter unter Überdruck stehenden Gasen das Ausströmen erlauben, um ein Platzen der Behälter zu vermeiden. Selbstverständlich muß in diese Öffnung ein geeigneter Filter, z. B. ein Aktivkohlefilter, eingesetzt sein, der verhindert, daß Keime und dergleichen aus dem Kunststoffbehälter heraus gelangen können.

Die Kunststoffbehälter können auch aus einem insbesondere unter Wärmeeinwirkung dehnbaren Material ausgebildet sein. Dadurch kann ein bei der Erwärmung ihres Inhalts im Mikrowellenfeld auftretender Überdruck aufgefangen werden, ohne daß die Gefahr besteht, daß die Kunststoffbehälter unter dem Einfluß dieses Überdrucks platzen oder sonstwie zerstört werden könnten.

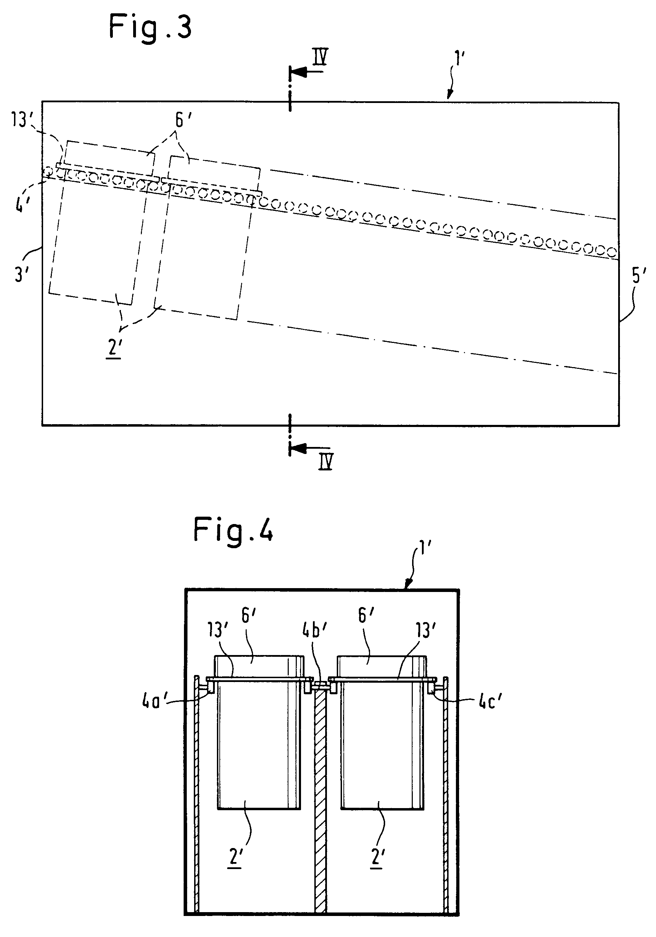

Fig. 3 zeigt schematisch eine weitere Ausführungsform bzw. eine Seitenansicht eines Sterilisierungstunnels, der allgemein mit 1′ bezeichnet ist. Der Sterilisierungstunnel 1′ besitzt ein Eintragende 3′ und ein Austragende 5′. Zwischen dem Eintragende 3′ und dem Austragende 5′ ist eine Rollenförderbahn 4′ so angeordnet, daß sie vom Eintragende zum Austragende hin leicht abwärts geneigt verläuft.

Bei dem gezeigten Ausführungsbeispiel kan am Eintragende 3′ eine (nicht gezeigte) Tür geöffnet werden und es werden dann die einzelnen Müllbehälter 2′ mit auf diesen aufgesetzten Deckeln 6′ so auf die Rollenförderbahnen 4′ aufgesetzt, daß die Müllbehälter an seitlichen Flanschen 13′ auf gegenüberliegenden Seiten durch Rollen abgestützt gehalten werden. Die abwärts gerichtete Neigung der Rollenförderbahn ist dabei so bemessen, daß die jeweiligen Müllbehälter aufgrund ihres Eigengewichts in Richtung zum Austragende 5′ gefördert werden, wobei sie über die Rollen der Rollenförderbahn 4′ gleiten. Am Austragende 5′ ist ein nicht gezeigter lösbarer Anschlag vorhanden, an dem der erste auf die Rollenförderbahn aufgesetzte Müllbehälter anstößt.

Nach einer anderen Ausführungsform (nicht gezeigt) könnte der Kunststoffbehälter mit einem Sack aus flexiblem Material versehen sein, der bei Erwärmung des Inhalts des Kunststoffbehälters im Mikrowellenfeld sich in den Raum außerhalb des Kunststoffbehälters ausstülpen kann und somit zum Abbau von Überdruck im Kunststoffbehälter beitragen kann.

Müllbehälter rollen in Richtung zum Austragende 5′ dann so weit nach, bis sie an den jeweils vorhergehenden Müllbehälter anstoßen, so daß dadurch eine durchgehende Reihe von Müllbehältern gebildet wird, zwischen denen keine Unterbrechung oder Zwischenraum vorhanden ist, so daß jeder Müllbehälter in einer exakten, immer gleichbleibenden Lage positioniert wird. Nachdem dann die Tür am Eintragende hermetisch verschlossen ist, wird in jeden Müllbehälter mit Hilfe einer Injektionseinrichtung (nicht gezeigt), die von oben in Richtung auf den jeweiligen Deckel eines Müllbehälters absenkbare Injektionsnadeln umfaßt, Wasser oder ein Desinfektionsmittel in jeden Müllbehälter injiziert.

Die Injektionsnadeln können dann entweder aus den jeweiligen Müllbehältern wieder zurückgezogen werden oder in eingestochenem Zustand gehalten werden, woraufhin eine Mikrowellen-Sterilisierungsanlage (nicht gezeigt) aktiviert wird. Aufgrund der dabei auftretenden Erwärmung des Inhaltes der Müllbehälter entwickeln sich Gase und Wasserdampf, so daß in jedem Müllbehälter ein Überdruck entsteht. Dieser Überdruck kann bei spielsweise mit Hilfe einer Druckmeßeinrichtung gemessen werden, die zweckmäßigerweise in das Leitungssystem der Wasserversorgung integriert sein kann.

Darüber hinaus kann eine Temperaturmeßeinrichtung vorgesehen sein, um die im Inneren der Müllbehälter jeweils auftretende Temperatur zu messen. Die Temperaturmeßeinrichtung umfaßt einen Temperaturfühler, wobei gemäß einer vorteilhaften Ausgestaltung der Erfindung die Injektionsnadel selbst als Temperaturfühler ausgebildet sein kann oder gemäß einer alternativen Ausführungsform im Hohlraum der Injektionsnadel ein verschiebbarer Temperatursensor angeordnet sein kann, der aus der Spitze der Injektionsnadel heraus bewegt werden kann, um die Temperatur im Inneren des jeweiligen Behälters zu messen.

Nach dem Ende des Sterilisierungsvorganges werden dann die eingestochenen Injektionsnadeln wieder zurückgezogen und es kann dann die gesamte Ladung des Sterilisierungstunnels durch Öffnen einer entsprechenden Tür am Austragende 5′ entleert werden.

Bei der in Fig. 3 gezeigten Ausführungsform können mehrere Rollenförderbahnen parallel nebeneinander verlaufend vorgesehen sein, also beispielsweise zwei Rollenförderbahnen, so daß jeweils zwei Müllbehälter nebeneinander liegend durch den Sterilisierungstunnel hindurch gefördert werden können.

Fig. 4 zeigt eine Schnittdarstellung gemäß der Linie I-I in Fig. 3.

Wie sich der Fig. 4 entnehmen läßt, sind insgesamt drei Rollenförderbahnen 4a′, 4b′ und 4c′ parallel nebeneinander verlaufend angeordnet. Es lassen sich daher auf die Rollenförderbahn 4a′ und 4b′ ein linker Müllbehälter 2′ und auf die Rollenförderbahn 4b′ und 4c′ ein rechter Müllbehälter aufsetzen, die dann parallel durch den Sterilisierungstunnel 1′ hindurchgefördert werden.

Der Sterilisierungstunnel kann zweckmäßigerweise auf einem Fahrgestell installiert sein, beispielsweise auf einem Lastwagen oder einem Lastwagenanhänger, wobei die gezeigte Konstruktion hierfür besonders geeignet ist, da sie in vergleichsweise kurzer und gedrängter Bauweise ausgeführt werden kann.

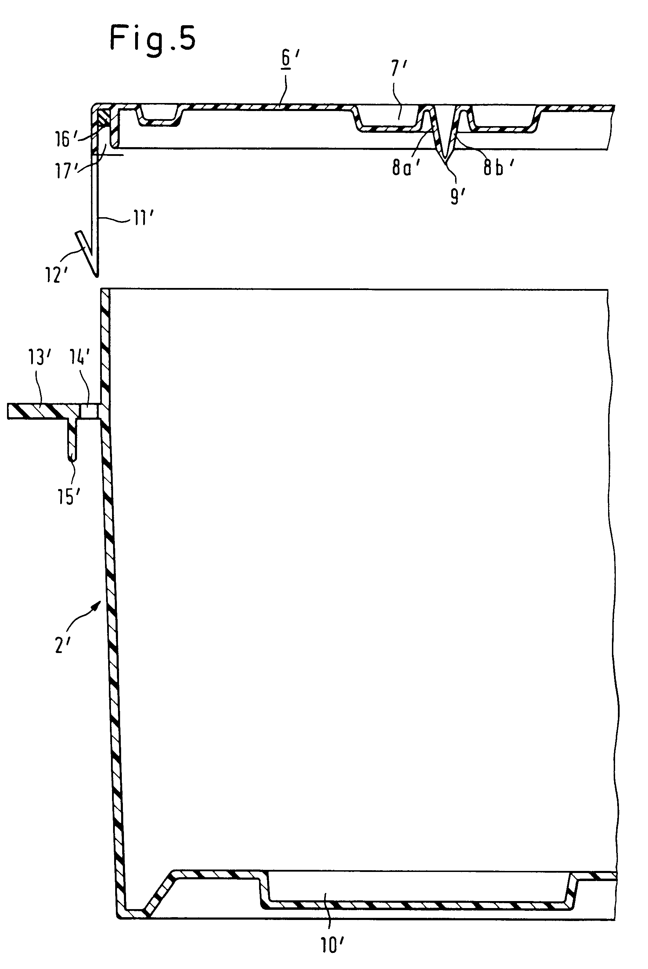

Fig. 5 zeigt schematisch eine Schnittdarstellung des Müllbehälters mit Deckel. Gemäß Fig. 5 ist ein Müllbehälter an seinem oberen Endbereich mit einem umlaufenden Flansch 13′ ausgestattet, der sich in radialer Richtung bzw. horizontal erstreckt. In dem umlaufenden Flansch sind in Abständen schlitzförmige Öffnungen 14′ ausgebildet, die Verriegelungszungen 11′, 12′ eines Deckels 6′ zugeordnet sind.

Wenn der in Fig. 5 gezeigte Behälter 2′ mit Müll gefüllt ist, wird er mit Hilfe des Deckels 6′ hermetisch verschlossen. Zu diesem Zweck braucht der Deckel 6′ lediglich über den oberen Rand des Behälters 2′ aufgesetzt und fest auf den oberen Rand gedrückt werden. Dabei dringen die Verriegelungszungen 11′, 12′ durch die schlitzförmigen Öffnungen 14′ im umlaufenden Flansch 13′ ein, durchdringen diese schlitzförmigen Öffnungen 14′ und erreichen schließlich eine Endstellung, in welcher ein hakenförmiger Vorsprung 12′ über einen von der unteren Fläche des umlaufenden Flansches 13′ abstehenden wandförmigen Vorsprung 15′ greifen, wodurch der Deckel fest mit dem Behälter 2′ verriegelt wird.

Der Deckel 6′ ist ferner mit einer umlaufenden Nut 17′ ausgestattet, in deren Bodenbereich eine Dichtung 16′ eingelegt ist, die aus einem elastischen Material wie beispielsweise Gummi besteht.

Beim Aufpressen des Deckels 6′ auf den oberen Rand des Behälters 2′ wird diese Dichtung 16′ elastisch verformt, so daß der hakenförmige Vorsprung 12′ über den wandförmigen Vorsprung 15′ rutschen kann, um dadurch die Verriegelung zu bewirken.

Der Behälter ist dann fest und insbesondere mediumsdicht verschlossen.

In einem zentralen Bereich des Deckels 6′ ist ferner eine Vertiefung 7′ ausgebildet, in deren zentralem Bereich ein Wandabschnitt gemäß einer Soll-Einstichstelle vorhanden ist. Dieser Wandabschnitt besteht aus zwei sich keilförmig verengenden Wänden 8a′ und 8b′, die eine keilförmige Vertiefung bilden.

Am Boden dieser Vertiefung gehen die beiden Wandabschnitte ineinander über bzw. sind einstückig miteinander verbunden. Dieser untere gemeinsame Wandabschnitt verjüngt sich nach unten zu, wie dies bei 9′ dargestellt ist. Dadurch ergibt sich ein relativ elastischer dünner Wandbereich, der die Soll-Einstichstelle bildet. Dadurch, daß die Wandabschnitte 8a′ und 8b′ keilförmig verlaufen, ergibt sich eine gewisse Führung der Injektionsnadel zum Bodenbereich 9′ der Vertiefung hin, so daß die Injektionsnadel genau zur Soll-Einstichstelle geführt wird.

Durch den in seiner Wandstärke verdünnten Wandfortsatz wird eine Art Rückschlagventil gebildet, wobei die Wirkungsweise dieser Konstruktion wie folgt ist:

Wenn eine Injektionsnadel durch den verlängerten Wandfortsatz gemäß der verdünnten Stelle 9′ hindurchgestoßen wird und anschließend wieder zurückgezogen wird, so werden die Wandabschnitte 8a′ und 8b′ im Bereich dieses verlängerten Wandfortsatzes bei sich allmählich erhöhendem Innendruck zusammengepreßt. Dadurch wird die Öffnung gemäß der Einstichstelle automatisch verschlossen, und zwar umso wirksamer je höher der Innendruck im Behälter steigt.

Gemäß einer alternativen Ausführungsform besteht die Möglichkeit, die Wandabschnitte 8a′ und 8b′ aus einem relativ weichen nachgiebigem Material herzustellen, wodurch das Verschließen der Einstichöffnung begünstigt wird.

Der Behälter 2′ kann in seinem Bodenbereich ferner eine Vertiefung 10′ aufweisen, die als zusätzliches Wasserreservoir dienen kann. Bei einer Erhitzung des im hermetisch verschlossenen Behälter enthaltenen Mülls und des injizierten Wassers wird zwangsläufig auch das Wasser in dem Wasserreservoir 10′ erhitzt und verdampft dabei, so daß der Müll im Inneren des Behälters intensiv mit Feuchtigkeit durchsetzt wird, so daß dadurch der Sterilisierungseffekt erhöht wird.

Dadurch, daß die einzelnen Müllbehälter im Sterilisierungstunnel so angeordnet werden, daß sie sich aneinander in Bewegungsrichtung abstützen, ergibt sich für jeden Müllbehälter eine exakte Positionierung innerhalb des Sterilisierungstunnels, so daß ein exaktes Einführen der Injektionsnadeln in den Bereich zwischen die Wandabschnitte 8a′ und 8b′ sichergestellt wird.

Die Erhitzung des Mülls im Inneren des Müllbehälters kann bis auf eine Temperatur von 150 °C gesteigert werden, wobei aber eine Temperatur von 120 °C bevorzugt wird.

Sowohl der Deckel 6′ als auch der Behälter 2′ können aus Polyethylen oder Polypropylen hergestellt sein.

Jeder Müllbehälter besitzt eine im wesentlichen quadratische Gestalt, wenn man von oben auf den Behälter blickt, so daß dadurch die exakte Positionierung des jeweiligen Behälters in dem Sterilisierungstunnel vollkommen unproblematisch ist.

Für den Fachmann sind eine Reihe von Abänderungen der erläuterten Ausführungsbeispiele ohne weiteres durchführbar, ohne jedoch dadurch den Rahmen der vorliegenden Erfindung zu verlassen. So besteht beispielweise die Möglichkeit, abhängig vom Meßergebnis beim Messen des Druckes im Inneren der Müllbehälter die Mikrowellenenergie zu regeln, um die Temperatur auf einem bestimmten vorgegebenen Wert zu halten bzw. den Druck im Inneren der Müllbehälter auf einem vorgegebenen Wert zu halten oder um bei Erreichen von vorbestimmten Druckwerten und Temperaturwerten die Erregung des Mikrowellengeneratorsystems (nicht gezeigt) abzuschalten.

Es besteht ferner auch die Möglichkeit, die Müllbehälter nicht quadratisch, sondern länglich bzw. rechteckig auszubilden, wobei auch bei dieser Gestalt eine hohe Sicherheit bei der Positionierung der Müllbehälter im Inneren des Sterilisierungstunnels erreicht wird, da die Müllbehälter dann nur in einer ganz bestimmten Drehlage auf die Rollenförderbahnen aufgesetzt bzw. eingeführt werden können.

## Patentansprüche

1. Verfahren zur Sterilisation von Müll, insbesondere Krankenhausmüll, unter Verwendung von mehreren Müllbehältern, die nach Beladung mit dem Müll durch einen Deckel hermetisch verschlossen werden und dann vorzugsweise zu Mehreren in einen Sterilisierungstunnel eingebracht werden und anschließend mit Mikrowellen bestrahlt werden, wobei entweder vor oder während der Mikrowellenbestrahlung Wasser oder ein Desinfektionsmittel in den Müll der Müllbehälter eingebracht wird und der oder die Müllbehälter dann an einem Austragende aus dem Sterilisierungstunnel ausgetragen werden,
**dadurch gekennzeichnet**, daß
a) die mit Müll beladenen und mit einem Deckel hermetisch verschlossenen Müllbehälter (6) auf ein Vorbereitungsförderband (5) aufgebracht werden, welches in einer Schleusenkammer (2) des Sterilisierungstunnels angeordnet ist,
b) die Schleusenkammer (2) dann hermetisch am Eingabeende (14) verschlossen wird,
c) eine Schleusentür (7) innerhalb des Sterilisierungstunnels geöffnet wird und die Müllbehälter (6) von dem Vorbereitungsförderband (5) auf ein Hauptförderband (8) innerhalb einer Sterilisierungskammer (3) aufgebracht werden,
d) die Sterilisierungskammer (3) dann hermetisch verschlossen wird und über bewegbar in der Sterilisierungskammer (3) angeordnete Injektionsnadeln (17) Wasser oder Desinfektionsmittel in jeden einzelnen Müllbehälter (6) injiziert wird, und
e) nach der Mikrowellenbehandlung alle in der Sterilisierungskammer (3) vorhandenen Müllbehälter (6) aus der Sterilisierungskammer am Austragende mit Hilfe des Hauptförderbandes (8) ausgetragen werden, wobei die Schleusentür (7) hermetisch verschlossen gehalten wird.

2. Verfahren nach Anspruch 1,
dadurch gekennzeichnet,
daß die Injektionsnadeln (17) in der Sterilisierungskammer (3) von oben nach unten auf die jeweiligen Müllbehälter (6) abgesenkt werden, um den jeweiligen Deckel der Müllbehälter (6) zu durchstechen.

3. Verfahren nach Anspruch 1,
dadurch gekennzeichnet,
daß die Injektionsnadeln (17) in der Sterilisierungskammer (3) von der Seite her auf die Müllbehälter (6) zu bewegt werden, um die Müllbehältr (6) seitlich zu durchstechen.

4. Verfahren nach Anspruch 2 oder 3,
dadurch gekennzeichnet,
daß die Injektionsnadeln (17) während der Mikrowellenbehandlung in ihrem Einstichzustand gehalten werden.

5. Verfahren nach einem der Ansprüche 1 bis 4,
dadurch gekennzeichnet,
daß mehr als eine Injektionsnadel (17) pro Müllbehälter (6) eingestochen werden.

6. Verfahren nach Anspruch 5,
dadurch gekennzeichnet,
daß eine der Injektionsnadeln (17) zum Injizieren von Wasser oder Desinfektionsmittel verwendet wird, während wenigstens eine zweite Injektionsnadel (17) zum Absaugen von Gasen aus dem Müllbehälter (6) verwendet wird.

7. Verfahren nach Anspruch 6,
dadurch gekennzeichnet,
daß das abgesaugte Gas über einen Sterilisierungskreislauf geschickt wird.

8. Verfahren nach einem der Ansprüche 2 bis 6,
dadurch gekennzeichnet,
daß wenigstens eine der Injektionsnadeln (17) dazu verwendet wird, um vorzugsweise erhitzten Wasserdampf in den Müllbehälter zu injizieren.

9. Verfahren nach Anspruch 6,
dadurch gekennzeichnet,
daß das abgesaugte Gas über ein Filter, einen Erhitzungsbereich und/oder einen Mikrowellenstrahlungsbereich geleitet wird.

10. Verfahren nach Anspruch 1,
dadurch gekennzeichnet,
daß die Schleusentür (7) hermetisch verriegelt wird, wenn die Austragstür (12) der Sterilisierungskammer (3) oder die Eintragstür (4) der Schleusenkammer (2) geöffnet wird und daß die Schleusentür (7) nur dann entriegelt wird, wenn die Austragstür (12) der Sterilisierungskammer (3) geschlossen ist und die Eintragstür (4) der Schleusenkammer (2) geschlossen wird.

11. Vorrichtung zur Durchführung des Verfahrens nach einem der Ansprüche 1 bis 10, mit einem Sterilisierungstunnel, der eine Sterilisierungskammer mit einem Eintragende und einem Austragende und einem Förderband aufweist,
**dadurch gekennzeichnet**, daß
a) die Sterilisierungskammer (3) am Eintragende mit dem Austragende einer Schleusenkammer (2) verbunden ist,
b) in der Schleusenkammer (2) ein Vorbereitungsförderband (5) angeordnet ist, welches so lang bemessen ist, daß mehrere Müllbehälter (6) in Förderrichtung auf dem Förderband (5) aufgestellt werden können,
c) die Schleusenkammer (2) gegenüber der Sterilisierungskammer (3) duch eine Schleusentür (7) hermetisch verschließbar ist, und
d) ein Mechanismus vorgesehen ist, durch den die Schleusentür (7) nur dann geöffnet wird, wenn die die Austragöffnung der Sterilisierungskammer (3) verschließende Tür (12) geschlossen und auch die die Eintragöffnung der Schleusenkammer (2) verschließende Tür (4) geschlossen ist.

12. Vorrichtung nach Anspruch 11, gekennzeichnet durch einen Mechanismus zum hermetischen Verschließen der die Austragöffnung der Sterilisierungskammer (3) verschließenden Tür (12), wenn die Schleusentür (7) geöffnet ist.

13. Vorrichtung nach Anspruch 11 oder 12,
dadurch gekennzeichnet,
daß in der Sterilisierungskammer (3) ein Mechanismus zum Absenken von in Abständen zueinander angeordneten Injektionsnadeln (17) vorgesehen ist.

14. Vorrichtung nach Anspruch 11,
dadurch gekennzeichnet,
daß in der Sterilisierungskammer (3) ein Mechanismus zum Bewegen von in Abständen angeordneten Injektionsnadeln in horizontaler Richtung vorgesehen ist.

15. Vorrichtung nach Anspruch 13 oder 14,
dadurch gekennzeichnet,
daß der Abstand der Injektionsnadeln (17) im wesentlichen dem Abstand der Müllbehälter (6) auf dem Hauptförderband (8) entspricht.

16. Vorrichtung nach Anspruch 15,
dadurch gekennzeichnet,
daß jeweils einem Müllbehälter (6) mehrere Injektionsnadeln (17) zugeordnet sind.

17. Vorrichtung nach einem der Ansprüche 11 bis 16, gekennzeichnet durch eine Einrichtung, um in die Sterilisierungskammer (3) erhitzten Dampf unter Druck einzuleiten, und durch eine Einrichtung, um in der Sterilisierungskammer einen Unterdruck zu erzeugen.

18. Vorrichtung nach einem der Ansprüche 11 bis 17,
dadurch gekennzeichnet,
daß das Vorbereitungsförderband (5) und das Hauptförderband (8) eine solche Breite haben, daß mehrere Müllbehälter (6) nebeneinander auf das jeweilige Förderband (5, 8) aufgestellt werden können.

19. Vorrichtung nach Anspruch 18,
dadurch gekennzeichnet,
daß das Vorbereitungsförderband (5) und das Hauptförderband (8) vom Eintragende zum Austragende hin jeweils schräg nach oben verlaufend angeordnet sind.

20. Vorrichtung nach einem der Ansprüche 11 bis 19,
dadurch gekennzeichnet,
daß die Schleusenkammer (2) und die Sterilisierungskammer (3) in einem transportierbaren Container (1) ausgebildet sind.

21. Vorrichtung nach einem der Ansprüche 11 bis 20, gekennzeichnet durch einen am Müllbehälter (6) angebrachten Code und durch eine Code-Leseeinrichtung am Eingabebereich der Schleusenkammer (2), um für die Sterilisationsanlage geeignete Müllbehälter (6) zu erkennen und um nicht geeignete Müllbehälter abzuweisen.

22. Vorrichtung nach einem der vorhergehenden Ansprüche,
dadurch gekennzeichnet,
daß Injektionsnadeln verwendet werden, die mehrere Kanäle mit Öffnungen besitzen, die vorzugsweise im Abstand voneinander liegen, um Flüssigkeiten und/oder Gase in die Müllbehälter ein- und/oder ausströmen zu lassen.

23. Einrichtung zur Sterilisierung von Müll, insbesondere Krankenhausmüll, mit mehreren, hermetisch durch einen Deckel verschließbaren und von einer Injektionsnadel durchstoßbaren Müllbehältern, die für die Sterilisierung von in ihnen enthaltenem Müll in einem Mikrowellen-Sterilisierungstunnel geeignet sind, welche wenigstens eine Förderbahn zum gleichzeitigen Fördern von mehreren hintereinander angeordneten Müllbehältern enthält, und welcher eine Injektionseinrichtung enthält, um in eine Reihe von Müllbehältern vorzugsweise gleichzeitig Wasser oder ein Desinfektionsmittel zu injizieren,
**dadurch gekennzeichnet**, daß
a) die wenigstens eine Förderbahn aus einer Rollenförderbahn (4′) gebildet ist, die von dem Eintragende (3′) zum Austragende (5′) des Sterilisierungstunnels (1′) hin abwärts geneigt ist,
b) die Müllbehälter (2′, 6′) in der Nähe ihrer Oberseite jeweils mit einem umlaufenden Flansch (13′) versehen sind, mit dessen Hilfe die Müllbehälter auf die wenigstens eine Rollenförderbahn (4′) aufgesetzt und von dieser getragen werden, und
c) die Müllbehälter (2′, 6′) mit einem eine Soll-Einstichstelle festlegenden und den Einstich einer Injektionsnadel verschließenden Wandabschnitt (7′, 8a′, 8b′, 9′) versehen sind.

24. Einrichtung nach Anspruch 23,
dadurch gekennzeichnet,
daß in dem Sterilisierungstunnel zwei Rollenförderbahnen (4a′, 4b′, 4c′) parallel nebeneinander verlaufend angeordnet sind.

25. Einrichtung nach Anspruch 23 oder 24,
dadurch gekennzeichnet,
daß am Austragende (5′) der jeweiligen Rollenförderbahn ein lösbarer Anschlag vorgesehen ist, bis zu welchem der erste in den Sterilisierungstunnel (1′) eingetragene Müllbehälter (2′, 6′) rollen kann.

26. Einrichtung nach Anspruch 23 oder 25,
dadurch gekennzeichnet,
daß die Neigung der jeweiligen Rollenförderbahn (4′) derart bemessen ist, daß die in der jeweiligen Rollenförderbahn aneinander gereihten Müllbehälter (2′, 6′) aufgrund ihres Eigengewichtes in Richtung zum Austragende (5′) des Sterilisierungstunnels (1′) rollen, wobei der vorderste Müllbehälter an dem lösbaren Anschlag abgestützt wird und sich die nachfolgenden Müllbehälter aneinander abstützen, so daß eine durchgehende, nicht unterbrochene Behälterreihe gebildet wird.

27. Einrichtung nach einem der Ansprüche 23 bis 26,
dadurch gekennzeichnet,
daß am Eintragende (3′) des Sterilisierungstunnels (1′) eine Schleusenkammer angeordnet ist.

28. Einrichtung nach einem der Ansprüche 23 bis 27, gekennzeichnet durch ein Querförderband, welches am Austragende des Sterilisierungstunnels (1′) angeordnet ist, um die ausgetragenen Müllbehälter (2′, 6′) zu einem Preßmüllcontainer zu fördern.

29. Einrichtung nach einem der Ansprüche 23 bis 28,
dadurch gekennzeichnet,
daß der Sterilisierungstunnel (1′) verfahrbar und in einem Lastwagen oder Lastwagenanhänger untergebracht ist.

30. Einrichtung nach Anspruch 23,
dadurch gekennzeichnet,
daß der Müllbehälter in Draufsicht etwa quadratisch oder rechteckig gestaltet ist und abgerundete Ecken aufweist.

31. Einrichtung nach Anspruch 23 oder 30,
dadurch gekennzeichnet,
daß der umlaufende Flansch (13′) in Abständen voneinander angeordnete Durchbrüche (14′) aufweist, durch welche am Deckel (6′) des Müllbehälters ausgebildete Verriegelungszungen (11′, 12′) einschiebbar sind, um den Deckel (6′) an dem umlaufenden Flansch (13′) zu verriegeln.

32. Einrichtung nach Anspruch 31,
dadurch gekennzeichnet,
daß der Deckel eine den oberen Behälterrand aufnehmende umlaufende Nut (17′) aufweist, in der ein Dichtungselement (16′) zur Abdichtung des Deckels am Behälter angeordnet ist.

33. Einrichtung nach Anspruch 32,
dadurch gekennzeichnet,
daß das Dichtungselement (16′) aus einem elastischen Material wie zum Beispiel Gummi besteht.

34. Einrichtung nach einem der Ansprüche 23, 30 bis 33,
dadurch gekennzeichnet,
daß der die Soll-Einstichstelle festlegende Wandabschnitt am Deckel (6′) des Müllbehälters ausgebildet ist und aus einer sich keilförmig verengenden Wandvertiefung (8a′, 8b′) besteht, wobei die Berührungslinie der Wandabschnitte (8a′, 8b′) am Boden der Vertiefung die Einstichstelle bildet.

35. Einrichtung nach Anspruch 34,
dadurch gekennzeichnet,
daß die am Boden der Vertiefung sich berührenden Wandabschnitte (8a′, 8b′) einstückig miteinander verbunden sind.

36. Einrichtung nach Anspruch 35,
dadurch gekennzeichnet,
daß die keilförmig zusammenlaufenden Wandabschnitte (8a′, 8b′) in einen zum Inneren des Behälters hin verlängerten Wandfortsatz übergehen.

37. Einrichtung nach Anspruch 36,
dadurch gekennzeichnet,
daß der Wandfortsatz in einen verdünnten Wandfortsatzrand (9′) ausläuft.

38. Einrichtung nach einem der Ansprüche 34 bis 37,
dadurch gekennzeichnet,
daß der die Soll-Einstichstelle festlegende Wandabschnitt (8a′, 8b′, 9′) in einer Vertiefung (7′) des Deckels (6′) oder der Seitenwand des Behälters (2′) ausgebildet ist.

39. Einrichtung nach einem der Ansprüche 30 bis 38,
dadurch gekennzeichnet,
daß im Boden des Müllbehälters eine als Wasserreservoir dienende Vertiefung (10′) ausgebildet ist.

40. Einrichtung nach einem der Ansprüche 23 bis 39, gekennzeichnet durch eine mit der Injektionsnadel verbundene Druck- und Temperaturmeßeinrichtung.

41. Einrichtung nach Anspruch 40,
dadurch gekennzeichnet,
daß die Druckmeßeinrichung in dem Leitungssystem der Wasserversorgung integriert ist.

42. Einrichtung nach Anspruch 41,
dadurch gekennzeichnet,
daß die Druckmeßeinrichtung zentral am Wasserverteilungssystem vorgesehen ist, wobei eine Wasserhauptleitung vorhanden ist, von der sich die einzelnen Wasserzuleitungen zu den Injektionsnadeln abzweigen.

43. Einrichtung nach Anspruch 40,
dadurch gekennzeichnet,
daß die Injektionsnadel selbst als Temperaturfühler ausgebildet ist.

44. Einrichtung nach Anspruch 40,
dadurch gekennzeichnet,
daß in dem Hohlraum der Injektionsnadel eine Temperaturmeßsonde verschiebbar angeordnet ist.

45. Einrichtung nach einem der Ansprüche 30 bis 39,
dadurch gekennzeichnet,
daß der Deckel und/oder der Behälter aus Polyethylen oder Polypropylen bestehen.

## Claims

1. A process for the sterilisation of refuse, especially hospital refuse, by using several refuse containers, which after being filled with refuse are hermetically sealed by a lid and then are introduced preferably several at a time into a sterilising tunnel and are then exposed to microwaves, with water or a disinfectant being introduced into the refuse in the refuse containers either before or during the microwave treatment and the refuse container or containers then being discharged from the sterilising tunnel at a delivery end,
**characterised in that**
a) the refuse containers (6) charged with refuse and hermetically sealed with a lid can be deposited on a lead-up conveyor belt (5), which is provided in a sluice chamber (2) of the sterilising tunnel,
b) the sluice chamber (2) is then hermetically sealed at the charging end (14)
c) a sluice door (7) inside the sterilising tunnel is opened and the refuse containers (6) are deposited from the lead-up conveyor belt (5) onto a main conveyor belt (8) inside a sterilising chamber (3),
d) the sterilising chamber (3) is then hermetically sealed and water or disinfectant is injected into each individual refuse container (6) via injection needles (17) movably disposed in the sterilising chamber (3) and
e) after the microwave treatment all refuse containers (6) present in the sterilising chamber (3) are discharged from the sterilising chamber at the delivery end by means of the main conveyor belt (8), with the sluice door (7) being kept hermetically sealed.

2. A process according to Claim 1,
**characterised in that** the injection needles (17) in the sterilising chamber (3) are lowered from above onto the respective refuse containers (6) in order to pierce the respective lid of the refuse containers (6).

3. A process according to Claim 1,
**characterised in that** the injection needles (17) in the sterilising chamber (3) are moved from the side towards the refuse container (6) in order to laterally pierce the refuse containers (6).

4. A process according to Claim 2 or 3,
**characterised in that** the injection needles (17) are kept in their inserted state during the microwave treatment.

5. A process according to one of Claims 1 to 4,
**characterised in that** more than one injection needle (17) is inserted per refuse container (6).

6. A process according to Claim 5,
**characterised in that** one of the injection needles (17) is used to inject water or disinfectant, while at least a second injection needle (17) is used to aspirate gases from the refuse container (6).

7. A process according to Claim 6,
**characterised in that** the aspirated gas is delivered via a sterilising circulation.

8. A process according to one of Claims 2 to 6,
**characterised in that** at least one of the injection needles (17) is used in order to inject preferably heated water vapour into the refuse container.

9. A process according to Claim 6,
**characterised in that** the aspirated gas is conveyed over a filter, a heating region and/or a microwave treatment region.

10. A process according to Claim 1,
**characterised in that** the sluice door (7) is hermetically locked when the delivery door (12) of the sterilising chamber (3) or the charging door (4) of the sluice chamber (2) is opened,
**and in that** the sluice door (7) is only unlocked when the delivery door (12) of the sterilising chamber (3) is closed and the charging door (4) of the sluice chamber (2) is closed.

11. An apparatus for performing the process according to one of Claims 1 to 10, having a sterilising tunnel, which comprises a sterilising chamber having a charging end and a delivery end and a conveyor belt, **characterised in that**
a) the sterilising chamber (3) at the charging end is connected to the delivery end of a sluice chamber (2),
b) in the sluice chamber (2) a lead-up conveyor belt (5) is provided, which is sufficiently long for several refuse containers (6) to be erected on the conveyor belt (5) in the conveying direction,
c) the sluice chamber (2) can be hermetically sealed in relation to the sterilising chamber (3) by a sluice door (7), and
d) a mechanism is provided by which the sluice door (7) is only opened when the door (12) shutting the delivery aperture of the sterilising chamber (3) is closed and also the door (4) shutting the charging aperture of the sluice chamber (2) is closed.

12. An apparatus according to Claim 11,
**characterised by** a mechanism for hermetically sealing the door (12) closing the delivery aperture of the sterilising chamber (3) when the sluice door (7) is opened.

13. An apparatus according to Claim 11 or 12,
**characterised in that** in the sterilising chamber (3) a mechanism for lowering injection needles (17) disposed at intervals from one another is provided.

14. An apparatus according to Claim 11,
**characterised in that** a mechanism is provided in the sterilising chamber (3) for moving injection needles (17) disposed at intervals from one another in the horizontal direction.

15. An apparatus according to Claim 13 or 14,
**characterised in that** the spacing of the injection needles (17) substantially corresponds to the spacing of the refuse containers (6) on the main conveyor belt (8).

16. An apparatus according to Claim 15,
**characterised in that** several injection needles (17) are associated with each refuse container (6).

17. An apparatus according to one of Claims 11 to 16,
**characterised by** a device for the introduction of heated steam under pressure into the sterilising chamber (3) in order to produce negative pressure in the sterilising chamber.

18. An apparatus according to one of Claims 11 to 17,
**characterised in that** the lead-up conveyor belt (5) and the main conveyor belt (8) have such a width that several refuse containers (6) can be placed next to one another on the respective conveyor belt (5, 8).

19. An apparatus according to Claim 18,
**characterised in that** the lead-up conveyor belt (5) and the main conveyor belt (8) are disposed so that they extend diagonally upwardly from the charging end to the delivery end.

20. An apparatus according to one of Claims 11 to 19,
**characterised in that** the sluice chamber (2) and the sterilising chamber (3) are constructed in a transportable container (1).

21. An apparatus according to one of Claims 11 to 20,
**characterised by** a code provided on the refuse container (6) and by a code reading device at the charging region of the sluice chamber (2) for recognising refuse containers (6) suitable for the sterilising installation and for rejecting unsuitable refuse containers.

22. An apparatus according to one of the preceding Claims,
**characterised in that** injection needles are used which have several ducts with apertures which are preferably spaced from one another in order to allow liquids and/or gases to flow into and/or out of the refuse containers.

23. An installation for sterilising refuse, especially hospital refuse, having several refuse containers which can be hermetically sealed by a lid and pierced by an injection needle, and which are suitable for the sterilisation of refuse contained therein in a microwave sterilising tunnel, which contains at least one conveyor for simultaneously conveying several refuse containers disposed one behind the other and which contains an injection device for the purpose of injecting water or a disinfectant into a row of refuse containers, preferably simultaneously,
**characterised in that**
a) at least one conveyor belt is formed from a gravity conveyor (4'), which is downwardly inclined from the charging end (3') to the delivery end (5') of the sterilising tunnel (1'),
b) in the vicinity of their upper sides the refuse containers (2', 6') are each provided with a circumferential flange (13'), by means of which the refuse containers are deposited on the gravity conveyor (4'), of which there is at least one, and are supported thereby, and
c) the refuse containers (2', 6') are provided with a wall section (7', 8a', 8b', 9') defining a predetermined injection site and closing the puncture hole of an injection needle.

24. An installation according to Claim 23,
**characterised in that** two gravity conveyors (4a', 4b', 4c') are provided in the sterilising tunnel so that they extend parallel next to one another.

25. An installation according to Claim 23 or 24,
**characterised in that** a detachable stop is provided at the delivery end (5') of the respective gravity conveyor, against which the first refuse container (2' 6') to be introduced into the sterilising tunnel (1') can roll.

26. An installation according to Claim 23 or 25,
**characterised in that** the inclination of the respective gravity conveyor (4') is such that the refuse containers (2', 6') lined up against one another in the respective gravity conveyor roll towards the delivery end (5') of the sterilising tunnel (1') because of their own weight, with the foremost refuse container being supported against the detachable stop and the following refuse containers being supported against one another, so that a continuous, uninterrupted row of containers is formed.

27. An installation according to one of Claims 23 to 26,
**characterised in that** a sluice chamber is provided at the charging end (3') of the sterilising tunnel (1').

28. An installation according to one of Claims 23 to 27,
**characterised by** a transverse conveyor belt, which is provided at the delivery end of the sterilising tunnel (1') for conveying the discharged refuse containers (2', 6') to a refuse container for compression.

29. An installation according to one of Claims 23 to 28,
**characterised in that** the sterilising tunnel (1') is mobile and is housed in a lorry or lorry trailer.

30. An installation according to Claim 23,
**characterised in that** in plan view the refuse container is roughly square or rectangular and has rounded corners.

31. An installation according to Claim 23 or 30,
**characterised in that** the circumferential flange (13') has openings (14') provided at intervals from one another, through which locking tongues (11', 12') constructed on the lid (6') of the refuse container can be inserted in order to lock the lid (6') on the circumferential flange (13').

32. An installation according to Claim 31,
**characterised in that** the lid has a circumferential groove (17') receiving the upper edge of the container, in which a sealing element (16') is provided for sealing the lid on the container.

33. An installation according to Claim 32,
**characterised in that** the sealing element (16') is made from an elastic material such as rubber, for example.

34. An installation according to one of Claims 23, 30 to 33,
**characterised in that** the wall section defining the predetermined insertion site is constructed on the lid (6') of the refuse container and consists of a wall depression (8a', 8b') narrowing in a wedge shape, with the line of contact of the wall sections (8a', 8b') at the base of the depression forming the insertion site.

35. An installation according to Claim 34,
**characterised in that** the wall sections (8a', 8b') touching one another at the base of the depression are connected to one another in one piece.

36. An installation according to Claim 35,
**characterised in that** the wall sections (8a', 8b') converging in a wedge shape pass into a wall extension lengthened towards the interior of the container.

37. An installation according to Claim 36,
**characterised in that** the wall extension ends in a reduced wall extension edge (9').

38. An installation according to one of Claims 34 to 37,
**characterised in that** the wall section (8a', 8b', 9') defining the predetermined insertion site is constructed in a depression (7') of the lid (6') or of the side wall of the container (2').

39. An installation according to one of Claims 30 to 38,
**characterised in that** a depression (10') used as a water reservoir is constructed in the base of the refuse container.

40. An installation according to one of Claims 23 to 39,
**characterised by** a pressure and temperature measurement device connected to the injection needle.

41. An installation according to Claim 40,
**characterised in that** the pressure measurement device is integrated in the pipe system of the water supply.

42. An installation according to Claim 41,
**characterised in that** the pressure measurement device is provided centrally on the water distribution system, a main water line being provided, from which the individual water supply lines to the injection needles branch off.

43. An installation according to Claim 40,
**characterised in that** the injection needle is itself formed as a temperature sensor.

44. An installation according to Claim 40,
**characterised in that** a temperature measurement probe is displaceably disposed in the cavity of the injection needle.

45. An installation according to one of Claims 30 to 39,
**characterised in that** the lid and/or the container are made from polyethylene or polypropylene.

## Revendications

1. Procédé pour la stérilisation de déchets, notamment de déchets d'hôpitaux, faisant appel à plusieurs bacs à déchets, lesquels sont fermés hermétiquement par un couvercle après leur remplissage de déchets, puis introduits, de préférence plusieurs à la fois, dans une galerie de stérilisation, à la suite de quoi ils sont exposés à des micro-ondes, de l'eau ou un désinfectant étant introduit dans les déchets des bacs à déchets, soit avant, soit après l'exposition aux micro-ondes, et le ou les bacs à déchets étant ensuite évacués de la galerie de stérilisation par une extrémité d'évacuation, procédé caractérisé en ce que :
a) les bacs à déchets (6) chargés de déchets et condamnés hermétiquement par un couvercle sont déposés sur une bande transporteuse de prétraitement (5) équipant un sas (2) de la galerie de stérilisation,
b) le sas (2) est ensuite fermé hermétiquement du côté chargement (14),
c) une porte de sas (7) s'ouvre à l'intérieur de la galerie de stérilisation, et les bacs à déchets (6) sont déposés par la bande transporteuse de prétraitement (5) sur une bande transporteuse principale (8) qui se trouve à l'intérieur d'une chambre de stérilisation (3),
d) la chambre de stérilisation (3) est ensuite fermée hermétiquement et de l'eau ou un désinfectant est injecté dans chaque bac à déchets (6) par des aiguilles d'injection (17) pouvant se déplacer à l'intérieur de la chambre de stérilisation (3), et
e) au terme du traitement aux micro-ondes, tous les bacs à déchets (6) présents à l'intérieur de la chambre de stérilisation (3) sont évacués de la chambre de stérilisation par le côté évacuation, au moyen de la bande transporteuse principale (8), la porte de sas (7) étant maintenue hermétiquement fermée.

2. Procédé selon la revendication 1, caractérisé en ce que les aiguilles d'injection (17) présentes dans la chambre de stérilisation (3) sont abaissées de haut en bas sur les bacs à déchets (6), pour transpercer les couvercles desdits bacs à déchets (6).

3. Procédé selon la revendication 1, caractérisé en ce que les aiguilles d'injection (17) présentes dans la chambre de stérilisation (3) sont dirigées latéralement vers les bacs à déchets (6), afin de transpercer les bacs à déchets (6) par le côté.

4. Procédé selon la revendication 2 ou 3, caractérisé en ce que les aiguilles d'injection (17) sont maintenues en situation de pénétration au cours du traitement aux micro-ondes.

5. Procédé selon l'une des revendications 1 à 4, caractérisé en ce que plus d'une aiguille d'injection (17) est enfoncée par bac à déchets (6).

6. Procédé selon la revendication 5, caractérisé en ce qu'une des aiguilles d'injection (17) est utilisée pour injecter de l'eau ou un désinfectant, tandis qu'au moins une deuxième aiguille d'injection (17) est utilisée pour extraire les gaz du bac à déchets (6).

7. Procédé selon la revendication 6, caractérisé en ce que le gaz extrait est amené à passer par un circuit de stérilisation.

8. Procédé selon l'une des revendications 2 à 6, caractérisé en ce qu'au moins une des aiguilles d'injection (17) est utilisée pour injecter dans le bac à déchets, de préférence, de la vapeur d'eau chauffée.

9. Procédé selon la revendication 6, caractérisé en ce que le gaz aspiré est amené à passer par un filtre, une zone de chauffe et/ou une zone d'irradiation aux micro-ondes.

10. Procédé selon la revendication 1, caractérisé en ce que la porte de sas (7) est verrouillée hermétiquement lorsque la porte d'évacuation (12) de la chambre de stérilisation (3) ou lorsque la porte de chargement (4) du sas (2) s'ouvre, et en ce que la porte de sas (7) n'est déverrouillée que si la porte d'évacuation (12) de la chambre de stérilisation (3) est fermée et si la porte de chargement (4) du sas (2) s'est refermée.

11. Dispositif pour la mise en oeuvre du procédé selon l'une des revendications 1 à 10, comportant une galerie de stérilisation, laquelle présente une chambre de stérilisation comprenant une extrémité chargement et une extrémité évacuation, et une bande transporteuse, caractérisé en ce que :
a) la chambre de stérilisation (3) communique, à l'extrémité chargement, avec l'extrémité évacuation d'un sas (2),
b) le sas (2) comporte une bande transporteuse de prétraitement (5), dont la longueur est telle que plusieurs bacs à déchets (6) peuvent être déposés sur la bande transporteuse (5) dans le sens de l'avancement,
c) le sas (2) peut être fermé hermétiquement par rapport à la chambre de stérilisation (3), par une porte de sas (7), et
e) il est prévu un mécanisme grâce auquel la porte de sas (7) ne s'ouvre que si la porte (12) qui condamne l'orifice d'évacuation de la chambre de stérilisation (3) et aussi la porte (4) qui condamne l'orifice de chargement du sas (2) sont fermées.

12. Dispositif selon la revendication 11, caractérisé par un mécanisme pour refermer hermétiquement la porte (12) qui condamne l'orifice d'évacuation de la chambre de stérilisation (3) lorsque la porte de sas (7) est fermée.

13. Dispositif selon la revendication 11 ou 12, caractérisé en ce qu'il est prévu, dans la chambre de stérilisation (3), un mécanisme permettant d'abaisser des aiguilles d'injection (17) espacées les unes des autres.

14. Dispositif selon la revendication 11, caractérisé en ce qu'il est prévu, dans la chambre de stérilisation (3), un mécanisme permettant de déplacer horizontalement des aiguilles d'injection espacées les unes des autres.

15. Dispositif selon la revendication 13 ou 14, caractérisé en ce que l'espacement des aiguilles d'injection (17) correspond, pour ainsi dire, à l'écartement des bacs à déchets (6) placés sur la bande transporteuse principale (8).

16. Dispositif selon la revendication 15, caractérisé en ce plusieurs aiguilles d'injection (17) sont affectées à un bac de déchets (6).

17. Dispositif selon l'une des revendications 11 à 16, caractérisé par un équipement permettant d'introduire, sous pression, de la vapeur chauffée dans la chambre de stérilisation (3), et par un équipement permettant de produire une dépression au sein de ladite chambre de stérilisation.

18. Dispositif selon l'une des revendications 11 à 17, caractérisé en ce que la bande transporteuse de prétraitement (5) et la bande transporteuse principale (8) ont une largeur telle que plusieurs bacs à déchets (6) peuvent être posés l'un à côté de l'autre sur chacune des bandes transporteuses (5, 8).

19. Dispositif selon la revendication 18, caractérisé en ce que la bande transporteuse de prétraitement (5) et la bande transporteuse principale (8) s'inclinent vers le haut, de l'extrémité chargement vers l'extrémité évacuation.

20. Dispositif selon l'une des revendications 11 à 19, caractérisé en ce que le sas (2) et la chambre de stérilisation (3) sont aménagés dans un conteneur transportable (1).

21. Dispositif selon l'une des revendications 11 à 19, caractérisé par un code appliqué sur le bac à déchets (6) et par un lecteur de code dans la zone de chargement du sas (2), pour détecter les bacs à déchets (6) aptes à pénétrer dans le stérilisateur, et pour rejeter ceux qui ne conviennent pas.

22. Dispositif selon l'une des revendications précédentes, caractérisé en ce que l'on utilise des aiguilles d'injection (17) comportant plusieurs passages avec des lumières, de préférence espacés les uns des autres, pour injecter des liquides et/ou des gaz dans les bacs à déchets (6) et/ou les en extraire.

23. Installation pour la stérilisation de déchets, notamment de déchets d'hôpitaux, comportant une pluralité de bacs à déchets pouvant être fermés hermétiquement par un couvercle et transpercés par une aiguille d'injection, ces bacs étant aptes à la stérilisation des déchets qu'ils contiennent dans une galerie de stérilisation à micro-ondes, laquelle comporte au moins une bande transporteuse permettant de transporter simultanément plusieurs bacs à déchets posés les uns à la suite des autres, et un dispositif d'injection, afin d'injecter de l'eau ou un désinfectant, de préférence en même temps, dans une rangée de bacs à déchets, caractérisée en ce que :
a) la bande transporteuse, au moins au nombre de un, est une bande transporteuse à rouleaux (4') qui s'incline vers le bas, partant de l'extrémité chargement (3') et allant vers l'extrémité évacuation (5') de la galerie de stérilisation (1'),
b) les bacs à déchets (2', 6') présentent, à proximité de leurs faces supérieures, un collet périphérique (13'), grâce auquel les bacs à déchets peuvent être déposés sur la bande transporteuse à rouleaux (4'), au moins au nombre de un, et portés par cette dernière, et
c) les bacs à déchets (2', 6') présentent une portion de paroi (7', 8a', 8b', 9') qui définit une zone de pénétration normalisée et obture la piqûre laissée par une aiguille d'injection.

24. Installation selon la revendication 23, caractérisée en ce que deux bandes transporteuses à rouleaux (4a', 4b', 4c') sont disposées en parallèle dans la galerie de stérilisation.

25. Installation selon la revendication 23 ou 24, caractérisée en ce qu'il est prévu, à l'extrémité évacuation (5') de chaque bande transporteuse à rouleaux, une butée amovible jusqu'à laquelle peut rouler le bac à déchets (2', 6') chargé en premier dans la galerie de stérilisation (1').

26. Installation selon la revendication 23 ou 25, caractérisée en ce que l'inclinaison des bandes transporteuses à rouleaux (4') est calculée de façon à ce que les bacs à déchets (2', 6') rangés les uns à côté des autres sur la bande transporteuse à rouleaux respective roulent en direction de l'extrémité évacuation (5') de la galerie de stérilisation (1'), par leur poids propre, le bac à déchets rangé le plus en avant venant s'appuyer sur la butée amovible, et les bacs à déchets suivants prenant appui les uns sur les autres, de telle sorte qu'il se forme une rangée de bacs continue et ininterrompue.

27. Installation selon l'une des revendications 23 à 26, caractérisée en ce qu'un sas est prévu à l'extrémité chargement (3') de la galerie de stérilisation (1').

28. Installation selon l'une des revendications 23 à 27, caractérisée par une bande transporteuse transversale, agencée à l'extrémité évacuation de la galerie de stérilisation (1'), pour acheminer les bacs à déchets (2', 6') à un conteneur pour le compactage des déchets.

29. Installation selon l'une des revendications 23 à 28, caractérisée en ce que la galerie de stérilisation (1') est déplaçable et montée dans un camion ou dans une remorque de camion.

30. Installation selon la revendication 23, caractérisée en ce que, vu d'en haut, le bac à déchets affecte une forme à peu près carrée ou à angles droits, et présente des coins arrondis.

31. Installation selon la revendication 23 ou 30, caractérisée en ce que le collet périphérique (13') présente des percements (14') espacés les uns des autres, dans lesquels viennent s'engager des languettes de verrouillage (11', 12') prévues sur le couvercle (6') du bac à déchets, pour verrouiller le couvercle (6') sur le collet périphérique (13').

32. Installation selon la revendication 31, caractérisée en ce que le couvercle présente une rainure périphérique (17') recevant le bord supérieur du bac, et dans laquelle est disposé un élément d'étanchéité (16') assurant l'étanchéité du couvercle sur le bac.

33. Installation selon la revendication 32, caractérisée en ce que l'élément d'étanchéité (16') est fabriqué dans une matière élastique, comme par exemple du caoutchouc.

34. Installation selon l'une des revendications 23, 30 à 33, caractérisée en ce que la portion de paroi définissant la zone de pénétration normalisée est ménagée sur le couvercle (6') du bac à déchets et constituée par un enfoncement de la paroi (8a', 8b') qui se resserre, en forme de coin, la ligne d'attouchement des portions de paroi (8a', 8b') au fond de l'enfoncement constituant la zone de pénétration.

35. Installation selon la revendication 34, caractérisée en ce que les portions de paroi (8a', 8b') au contact l'une de l'autre au fond de l'enfoncement sont rattachées d'un seul tenant.

36. Installation selon la revendication 35, caractérisée en ce que les portions de paroi (8a', 8b') qui convergent en forme de coin se transforment en une excroissance de paroi qui se prolonge vers l'intérieur du bac.

37. Installation selon la revendication 36, caractérisée en ce que l'excroissance de paroi se termine par un bord d'excroissance de paroi (9') aminci.

38. Installation selon l'une des revendications 34 à 37, caractérisée en ce que la portion de paroi (8a', 8b', 9') définissant la zone de pénétration normalisée est ménagée dans un enfoncement (7') du couvercle (6') ou de la paroi latérale du bac (2').

39. Installation selon l'une des revendications 30 à 38, caractérisée en ce qu'un enfoncement (10') servant de réservoir d'eau est ménagé au fond du bac à déchets.

40. Installation selon l'une des revendications 23 à 39, caractérisée par un dispositif de mesure de la pression et de la température relié à l'aiguille d'injection.

41. Installation selon la revendication 40, caractérisée en ce que le dispositif de mesure de la pression est intégré au système d'adduction de la distribution d'eau.

42. Installation selon la revendication 41, caractérisée en ce que le dispositif de mesure de la pression est disposé au centre du système de distribution d'eau, lequel comprend une canalisation d'eau principale dont dérivent des conduites de piquage individuelles desservant les aiguilles d'injection.

43. Installation selon la revendication 40, caractérisée en ce que l'aiguille d'injection proprement dite est configurée à la manière d'une sonde de température.

44. Installation selon la revendication 40, caractérisée en ce qu'une sonde de mesure de température est disposée, en pouvant coulisser, dans le creux de l'aiguille d'injection.

45. Installation selon l'une des revendications 30 à 39, caractérisée en ce que le couvercle et/ou le bac est/sont en polyéthylène ou en polypropylène.
